# EUROPÄISCHE PATENTSCHRIFT

(11) **EP 4 498 938 B1**
(45) Veröffentlichungstag und Bekanntmachung des Hinweises auf die Patenterteilung: **15.07.2026**
(21) Anmeldenummer: 23716173.2
(22) Anmeldetag: 30.03.2023
(51) Int. Cl.: A61B 17/16, A61B 17/00

(54) **MEDIZINISCHE WERKZEUGSPITZE, BETÄTIGUNGSVORRICHTUNG, CHIRURGISCHES INSTRUMENT UND BEFESTIGUNGSVERFAHREN**
MEDICAL TOOL TIP, ACTUATION DEVICE, SURGICAL INSTRUMENT, AND SECURING METHOD
POINTE D'OUTIL MÉDICAL, DISPOSITIF D'ACTIONNEMENT, INSTRUMENT CHIRURGICAL ET PROCÉDÉ DE FIXATION

(30) Priorität: 31.03.2022 DE 102022107751
(43) Veröffentlichungstag der Anmeldung: 05.02.2025
(73) Patentinhaber: Kammerer Medical Systems GmbH & Co.KG, 78333 Stockach (DE)
(72) Erfinder: SCHREMPP, Helena, 78576 Emmingen-Liptingen (DE); SCHWÄGLER, Rüdiger, 78576 Emmingen-Liptingen (DE)
(74) Vertreter: Heyerhoff Geiger GmbH & Co. KG
(86) Internationale Anmeldenummer: PCT/DE2023/100247
(87) Internationale Veröffentlichungsnummer: WO 2023/186219

(56) Entgegenhaltungen:
- DE-T2- 69 533 960
- DE-U1- 202019 104 247
- US-A1- 2014 100 593
- US-A1- 2019 000 488

## Beschreibung

Die vorliegende Erfindung betrifft eine medizinische Werkzeugspitze, eine Betätigungsvorrichtung, ein chirurgisches Instrument und ein Verfahren zur Befestigung einer medizinischen Werkzeugspitze an einer Betätigungsvorrichtung.

US 2019/000488 A1 offenbart eine medizinische Werkzeugspitze, eine Betätigungsvorrichtung und ein Verfahren zur Befestigung einer medizinische Werkzeugspitze an einer Betätigungsvorrichtung.

Bei einigen medizinischen Eingriffen werden chirurgische Instrumente verwendet, um Knochen, Knorpel oder anderes Gewebe zu entfernen oder zu entnehmen. Solche Instrumente weisen üblicherweise einen länglichen Schaft auf, an dessen distalem, d. h. dem Benutzer abgewandten, Ende ein medizinisches Werkzeug vorgesehen ist. Über einen Mechanismus am proximalen, d. h. dem Benutzer zugewandten, Ende des Schafts kann der Benutzer das Werkzeug, zum Beispiel eine Stanze oder eine Löffelzange, betätigen.

In der Praxis kommt es häufig vor, dass im Verlauf eines Eingriffs unterschiedliche Werkzeuge benötigt werden und/oder ein Werkzeug in mehreren Größen benötigt wird. Im Hinblick auf Anschaffungskosten, Lagerkapazität und Praktikabilität sind daher chirurgische Instrumente entwickelt worden, die aus einer Betätigungsvorrichtung und einer auswechselbaren medizinischen Werkzeugspitze bestehen. Je nach Bedarf können somit an einer einzigen Betätigungsvorrichtung, die vom Benutzer gehalten wird und am proximalen Ende den Mechanismus zur Betätigung des Werkzeugs aufweist, nacheinander die gewünschten Werkzeugspitzen befestigt werden. Dies kann auch die Sicherheit beim Eingriff durch sofortigen Ersatz bei Verbiegen oder Abstumpfung der Werkzeugspitze und die Nachhaltigkeit verbessern, da nicht das ganze Instrument, sondern nur die Spitze entsorgt werden muss.

Vor diesem Hintergrund ist es eine Aufgabe der vorliegenden Erfindung, die Benutzerfreundlichkeit beim Einsatz eines chirurgischen Instruments oder zumindest Teilen davon weiter zu verbessern.

Diese Aufgabe wird gelöst durch eine medizinische Werkzeugspitze, eine Betätigungsvorrichtung, ein chirurgisches Instrument sowie ein Verfahren zur Befestigung einer medizinischen Werkzeugspitze an einer Betätigungsvorrichtung gemäß den unabhängigen Ansprüchen.

Bevorzugte Ausführungsformen sind Gegenstand der abhängigen Ansprüche sowie der folgenden Beschreibung.

Ein erster Aspekt der Erfindung betrifft eine medizinische Werkzeugspitze für ein chirurgisches Instrument. Die Werkzeugspitze weist einen ersten, vorzugsweise einstückig ausgebildeten, Spitzenteil mit einem ersten Befestigungsabschnitt zur werkzeugfreien Befestigung des ersten Spitzenteils an einem Grundkörper einer Betätigungsvorrichtung und einen zweiten, vorzugsweise ein- oder zweistückig ausgebildeten, Spitzenteil mit einem zweiten Befestigungsabschnitt zur werkzeugfreien Befestigung des zweiten Spitzenteils an einem Schlitten der Betätigungsvorrichtung auf. Dabei ist der zweite Spitzenteil zumindest abschnittsweise längsverschiebbar am ersten Spitzenteil, insbesondere werkzeugfrei unlösbar, befestigt. Erfindungsgemäß sind der erste und zweite Befestigungsabschnitt derart ausgebildet, insbesondere derart relativ zueinander angeordnet oder durch Längsverschiebung anordenbar, dass eine Befestigung des zweiten Spitzenteils am Schlitten, insbesondere durch zumindest abschnittsweises Einschieben des zweiten Befestigungsabschnitts in den Schlitten, erst erfolgen kann, wenn der erste Spitzenteil bereits, insbesondere formschlüssig, am Grundkörper befestigt und dadurch gesichert ist.

Ein Aspekt der Erfindung beruht auf dem Ansatz, das Herstellen einer Verbindung zwischen einer medizinischen Werkzeugspitze mit einem ersten Spitzenteil und einem daran längsverschiebbar befestigten zweiten Spitzenteil und einer Betätigungsvorrichtung durch gattungstypisches Betätigen der Betätigungsvorrichtung zu ermöglichen. Dazu ist es einerseits zweckdienlich, dass (i) ein Schlitten der Betätigungsvorrichtung durch Längsverschiebung relativ zu einem Grundkörper der Betätigungsvorrichtung in eine Befestigungsstellung bringbar ist, in der sich der erste Spitzenteil am Grundkörper befestigen lässt, und (ii) der Schlitten - bei befestigtem ersten Spitzenteil - durch zumindest abschnittsweises Längsverschieben aus der Befestigungsstellung heraus mit dem zweiten Spitzenteil verbindbar ist. Andererseits ist es dazu auch zweckdienlich, dass ein erster Befestigungsabschnitt des ersten Spitzenteils zur Befestigung am Grundkörper und ein zweiter Befestigungsabschnitt des zweiten Spitzenteils zur Befestigung am Schlitten derart ausgebildet sind, insbesondere angeordnet oder anordenbar, beispielsweise derart in einer Längsrichtung der Werkzeugspitze voneinander beabstandet, dass eine Befestigung des zweiten Spitzenteils am Schlitten insbesondere auch dann möglich ist, nachdem der erste Befestigungsabschnitt bereits am Grundkörper befestigt wurde. Insofern ist es bevorzugt, dass der erste und zweite Befestigungsabschnitt derart ausgebildet sind, insbesondere angeordnet, beispielsweise beabstandet, dass die Werkzeugspitze zweistufig an der Betätigungsvorrichtung befestigbar ist.

Der Schlitten kann beispielsweise derart ausgebildet sein, dass er lediglich in der Befestigungsstellung ein Einführen des ersten Befestigungsabschnitts in einen Grundkörperbefestigungsabschnitt zur werkzeugfreien, insbesondere formschlüssigen, Befestigung am Grundkörper ermöglicht. Bei einer Befestigung des ersten Spitzenteils am Grundkörper wird der zweite Befestigungsabschnitt - vorzugsweise aufgrund seiner Anordnung relativ zum zweiten Befestigungsabschnitt - dabei zweckmäßigerweise noch nicht mit dem Schlitten verbunden. Durch die Befestigung am Grundkörper lässt sich das erste Spitzenteil jedoch vorzugsweise derart festlegen, dass sich ein Schlittenbefestigungsabschnitt bei einer Längsverschiebung des Schlittens - wie sie zweckmäßigerweise durch eine Betätigung der Betätigungsvorrichtung ausgelöst wird - auf den zweiten Befestigungsabschnitt schiebt und so werkzeugfrei eine, insbesondere formschlüssige, Verbindung auch zwischen dem Schlitten und dem zweiten Spitzenteil hergestellt wird.

Eine derartig ausgestaltete Befestigungsvorrichtung einerseits und/oder eine derartig ausgestaltete Werkzeugspitze andererseits ermöglicht nicht nur eine werkzeugfreie Befestigung der Werkzeugspitze an der Betätigungsvorrichtung, sondern auch eine Befestigung mit besonders wenigen Handgriffen. Eine gattungstypische Betätigung der Betätigungsvorrichtung zur Längsverschiebung des Schlittens aus der Befestigungsstellung heraus zur Verbindung mit dem zweiten Spitzenteil ist nämlich grundsätzlich einhändig möglich. Anders gesagt kann die Betätigungsvorrichtung mit einer Hand gehalten und auch mit dieser Hand betätigt - und damit die Werkzeugspitze vollständig an der Betätigungsvorrichtung befestigt - werden. Es ist insofern nicht nötig, einen gesonderten Verriegelungsmechanismus zu betätigen. Ohne einen solchen gesonderten Verriegelungsmechanismus besteht auch kein Risiko einer versehentlichen Auslösung während des Gebrauchs. Damit lassen sich verschiedene Werkzeugspitzen auch unter Stress und sterilen Bedingungen, zum Beispiel direkt in einem Operationssaal, schnell und sicher auswechseln.

Nachfolgend werden bevorzugte Ausführungsformen der Erfindung und deren Weiterbildungen beschrieben, die jeweils, soweit dies nicht ausdrücklich ausgeschlossen wird, beliebig miteinander sowie mit den im Weiteren beschriebenen Aspekten der Erfindung kombiniert werden können.

In einer bevorzugten Ausführungsform weisen der erste Befestigungsabschnitt und der zweite Befestigungsabschnitt zumindest abschnittsweise die gleiche Kontur auf. Anders gesagt sind der erste Spitzenteil im ersten Befestigungsabschnitt und der zweite Spitzenteil im zweiten Befestigungsabschnitt vorzugsweise derart ausgestaltet, dass sie zumindest abschnittsweise und aus einem vorgegebenen Blickwinkel denselben Umriss haben. Dabei müssen der erste und zweite Befestigungsabschnitt nicht zwingend die gleiche Bemaßung aufweisen. Insbesondere ist es denkbar, dass der erste Befestigungsabschnitt zumindest abschnittsweise kleiner, insbesondere schmaler, ausgebildet ist als der zweite Befestigungsabschnitt. Dies erlaubt eine einfache und intuitive Befestigung des ersten Befestigungsabschnitts am Grundkörper, da dadurch der Schlittenbefestigungsabschnitt, der eigentlich zur Verbindung des zweiten Spitzenteils eingerichtet ist, zur Führung des ersten Befestigungsabschnitts in den Grundkörperbefestigungsabschnitt dienen kann.

Es ist insofern bevorzugt, dass der zweite Befestigungsabschnitt quer zu einer Längserstreckung der Werkzeugspitze seitlich beidseitig über den ersten Befestigungsabschnitt hinausragt. Anders gesagt ist der erste Spitzenteil im ersten Befestigungsabschnitt schmaler ausgebildet als der zweite Spitzenteil im zweiten Befestigungsabschnitt. Dadurch kann sichergestellt werden, dass nur der erste Spitzenteil am Grundkörper befestigbar ist, nicht jedoch der zweite Spitzenteil. So kann insbesondere verhindert werden, dass die Stanzspitze falsch, insbesondere verkehrt herum, an der Betätigungsvorrichtung, d. h. der zweite Spitzenteil am Grundkörper, befestigt wird.

Alternativ zu der unterschiedlichen Dimensionierung, insbesondere Breite, des ersten und zweiten Befestigungsabschnitts sind aber auch andere Mittel zur Verhinderung einer falschen Befestigung denkbar. Beispielsweise kann der erste Spitzenteil auf einer dem zweiten Spitzenteil gegenüberliegenden Seite eine Nut zur Aufnahme einer Führungsschiene des Grundkörpers aufweisen. Dadurch sind der erste und zweite Befestigungsabschnitt nur dann vollständig von der Betätigungsvorrichtung aufnehmbar, wenn die Nut der Führungsschiene zugewandt ist. Insofern kann die Werkzeugspitze in einigen Ausführungsformen ganz allgemein dazu eingerichtet sein, eine falsche Befestigung an der Betätigungsvorrichtung zu verhindern.

Vorzugsweise weisen der erste und zweite Befestigungsabschnitt die gleiche Kontur in einer Kontaktebene auf. Es kann beispielsweise der erste Spitzenteil eine dem zweiten Spitzenteil zugewandte Oberseite mit einer ebenen ersten Kontaktfläche aufweisen. Entsprechend weist der zweite Spitzenteil zweckmäßigerweise eine dem ersten Spitzenteil zugewandte Unterseite mit einer ebenen zweiten Kontaktfläche auf. Mit der zweiten Kontaktfläche kann der zweite Spitzenteil die erste Kontaktfläche in zumindest einer Betriebsstellung kontaktieren. Vorzugsweise liegen die erste und zweite Kontaktfläche zumindest bei diesem Kontakt in der Kontaktebene. Die erste Kontaktfläche und die zweite Kontaktfläche sind vorzugsweise zumindest abschnittsweise gleichartig ausgeformt. Unter einer gleichartigen Ausformung kann hierbei insbesondere eine Ausformung der beiden Kontaktflächen verstanden werden, bei der die Kontaktflächen die gleiche Form aufweisen. Eine gleiche Dimensionierung, d. h. die gleichen Maße, sind nicht zwingend notwendig. Insbesondere kann die erste Kontaktfläche kleiner ausgebildet sein als die zweite Kontaktfläche. Mit derartig ausgebildeten Kontaktflächen kann die Konturengleichheit der beiden Befestigungsabschnitte hergestellt werden.

In einer weiteren bevorzugten Ausführungsform weist der erste Befestigungsabschnitt einen ersten Vorsprung auf, der sich längsverlaufend an ein proximales Ende des ersten Spitzenteils anschließt. Alternativ oder zusätzlich ist bevorzugt, dass der zweite Befestigungsabschnitt einen zweiten Vorsprung aufweist, der sich längsverlaufend an ein proximales Ende des zweiten Spitzenteils anschließt. Mithilfe solcher Vorsprünge ist eine besonders robuste Verbindung der Werkzeugspitze mit der Betätigungsvorrichtung möglich. Insbesondere kann dadurch verhindert werden, dass die Werkzeugspitze während des Gebrauchs aus der Betätigungsvorrichtung herausfällt, wenn diese um 180° um die Längsachse gedreht wird. Zudem kann so auch die Werkzeugspitze ohne Stufenbildung an die Betätigungsvorrichtung angeschlossen werden.

Der erste Vorsprung weist zweckmäßigerweise eine Oberseite mit einer ebenen Oberseitenfläche auf. Alternativ oder zusätzlich weist der zweite Vorsprung eine Unterseite mit einer ebenen Unterseitenfläche auf. In bevorzugter Weise ist die Unterseitenfläche im Wesentlichen parallel zur Oberseitenfläche ausgerichtet. Mit einer derartigen Ausgestaltung des ersten und zweiten Vorsprungs kann eine reibungslose Betätigung der Werkzeugspitze sichergestellt werden.

Eine besonders sichere und formschlüssige Befestigung der Werkzeugspitze an der Betätigungsvorrichtung lässt sich erreichen, indem der erste und zweite Vorsprung vorzugsweise jeweils Seitenwände aufweisen, die im Wesentlichen senkrecht zur Oberseitenfläche und der Unterseitenfläche verlaufen und gleichartig uneben ausgebildet sind.

Beispielsweise kann der erste Spitzenteil im ersten Befestigungsabschnitt eine Oberseite mit einer Oberseitenfläche und eine erste Ausbauchung aufweisen, die quer zu einer Flächennormalen der Oberseitenfläche verläuft. Alternativ oder zusätzlich kann der zweite Spitzenteil im zweiten Befestigungsabschnitt eine Unterseite mit einer der Oberseitenfläche zugewandte Unterseitenfläche und eine zweite Ausbauchung aufweisen, die quer zu einer Flächennormalen der Unterseitenfläche verläuft und gleichartig zur ersten Ausbauchung ausgeformt ist. Auch hier kann unter einer gleichartigen Ausformung insbesondere verstanden werden, dass die Ausformungen die gleiche Form, etwa die gleiche Kontur, aufweisen. Die Ausformungen müssen jedoch nicht zwingend gleich groß ausgebildet sein. Die Ausbauchungen erlauben eine formschlüssige Befestigung an der Betätigungsvorrichtung, insbesondere ein Hintergreifen durch den Grundkörperbefestigungsabschnitt bzw. den Schlittenbefestigungsabschnitt. Mithilfe der gleichartigen Ausformungen kann die Werkzeugspitze auch zuverlässig aus der Betätigungsvorrichtung entnommen werden. Werden diese im Wesentlichen fluchtend miteinander angeordnet, ist es möglich, auch den ersten Befestigungsabschnitt durch eine Ausnehmung im Schlitten - in welcher im Betrieb der zweite Befestigungsabschnitt angeordnet ist - herauszuführen.

Zweckmäßigerweise weisen der erste Spitzenteil und/oder der zweite Spitzenteil im Bereich der ersten bzw. zweiten Ausbauchung dabei jeweils eine konvexe Krümmung auf. Dadurch kann insbesondere der Schlitten im Schlittenbefestigungsabschnitt beim Aufschieben auf den zweiten Spitzenteil aufgebogen werden, sodass ein Teil des Schlittenbefestigungsabschnitts hinter der zweiten Ausbauchung einrastet.

In einer weiteren bevorzugten Ausführungsform ist die Werkzeugspitze als steriles Einwegwerkzeug ausgebildet. Dies kann die Logistik zum Beispiel in einem Krankenhaus erheblich vereinfachen, da die Werkzeugspitze nach einer Verwendung dann nicht sterilisiert werden muss. Zudem können dadurch immer neue und damit scharfe Werkzeugspitzen bereitgestellt werden. Durch die sichergestellte Sterilität eines solchen Einwegwerkzeugs kann darüber hinaus die Patientensicherheit erhöht werden.

Alternativ ist es aber auch möglich, dass die Werkzeugspitze wiederverwendbar, d. h. insbesondere reinigbar und/oder sterilisierbar, ausgebildet ist. Damit lassen sich gegebenenfalls der Beschaffungsaufwand und/oder Kosten verringern.

Ein zweiter Aspekt der Erfindung betrifft eine Betätigungsvorrichtung für ein chirurgisches Instrument. Die Betätigungsvorrichtung weist (i) einen Grundkörper mit einem Grundkörperbefestigungsabschnitt zur werkzeugfreien, insbesondere formschlüssigen, Befestigung eines ersten Spitzenteils einer medizinischen Werkzeugspitze am Grundkörper und (ii) einen Schlitten mit einem Schlittenbefestigungsabschnitt zur werkzeugfreien, insbesondere formschlüssigen, Befestigung eines am ersten Spitzenteil längsverschiebbar, insbesondere werkzeugfrei unlösbar, befestigten zweiten Spitzenteils der Werkzeugspitze am Schlitten auf. Dabei ist der Schlitten zumindest abschnittsweise längsverschiebbar am Grundkörper, insbesondere werkzeugfrei unlösbar, befestigt. Erfindungsgemäß ist zur werkzeugfreien Befestigung der Werkzeugspitze an der Betätigungsvorrichtung der Schlitten (i) durch zumindest abschnittsweises Längsverschieben relativ zum Grundkörper in eine Befestigungsstellung bringbar, in welcher der erste Spitzenteil im Grundkörperbefestigungsabschnitt am Grundkörper befestigbar ist. Der Schlitten ist zudem (ii) derart ausgebildet, dass der zweite Spitzenteil bei am Grundkörper befestigtem ersten Spitzenteil durch zumindest abschnittsweises Längsverschieben des Schlittens aus der Befestigungsstellung heraus im Schlittenbefestigungsabschnitt mit dem Schlitten verbindbar, insbesondere zumindest abschnittsweise in den Schlittenbefestigungsabschnitt einführbar, ist.

Die Betätigungsvorrichtung kann derart ausgebildet sein, dass der Schlitten in der Befestigungsstellung vom Grundkörper abgehoben ist. Anders gesagt kann sich beim Längsverschieben des Schlittens in die Befestigungsstellung ein Spalt zwischen dem Schlitten und dem Grundkörper bilden. Dabei sind Schlitten und Grundkörper zweckmäßigerweise - wie bei gattungstypischer Betätigung der Betätigungsvorrichtung - im Wesentlichen parallel zueinander ausgerichtet.

Eine derartige Betätigungsvorrichtung ist besonders benutzerfreundlich, da hiermit zum Beispiel ein schneller Wechsel verschiedener Werkzeugspitzen auch unter Stress und sterilen Bedingungen, etwa direkt im Operationssaal, möglich ist. Dies kann unter anderem zu kürzeren Operationszeiten führen. Zudem lässt sich mit der Betätigungsvorrichtung auch ein Platzbedarf auf einem Instrumentensieb reduzieren, da auch für verschiedene Anwendungen nun nicht mehr mehrere vollständige chirurgische Instrumente benötigt werden.

In einer bevorzugten Ausführungsform fluchten eine Kontur des Grundkörperbefestigungsabschnitts und eine Kontur des Schlittenbefestigungsabschnitts zumindest abschnittsweise und im Wesentlichen miteinander, wenn sich der Schlitten in der Befestigungsstellung befindet. Dies erlaubt es beispielsweise, den ersten Befestigungsabschnitt durch den Schlittenbefestigungsabschnitt hindurch in den Grundkörperbefestigungsabschnitt zur Befestigung des ersten Spitzenteils am Grundkörper zu führen.

Im Sinne der Erfindung fluchten zwei Bauteile oder deren Konturen hierbei insbesondere bereits dann zumindest abschnittsweise und im Wesentlichen, wenn die wesentlichen Merkmale der Bauteile bzw. Konturen, etwa Ecken oder Kanten oder sonstige markante Ausformungen, aneinander ausgerichtet sind. Unter einem Fluchten ist also gerade keine absolute Deckungsgleichheit zu verstehen. Vielmehr kann darunter eine Lage von zwei Bauteilen relativ zueinander verstanden werden, in der die Bauteile bzw. deren Konturen, insbesondere deren markante Abschnitte, miteinander korrespondieren. Insofern können zwei Bauteile zumindest abschnittsweise und im Wesentlichen auch dann miteinander fluchten, wenn das eine Bauteil geringfügig größer bemessen ist als das andere Bauteil.

Ein zumindest abschnittsweises Fluchten der Konturen des Grundkörperbefestigungsabschnitts und des Schlittenbefestigungsabschnitts im Wesentlichen ist zum Beispiel möglich, wenn der Grundkörper eine dem Schlitten zugewandte Grundkörperoberseite mit einer ebenen Grundkörpergleitfläche aufweist. Entsprechend kann der Schlitten eine dem Grundkörper zugewandte Schlittenunterseite mit einer ebenen Schlittengleitfläche aufweisen, mit welcher der Schlitten bei einer Längsverschiebung relativ zum Grundkörper zumindest abschnittsweise an der Grundkörpergleitfläche entlanggleitet, beispielsweise bis der Schlitten vom Grundkörper abhebt. Dies kann den reibungslosen Betrieb der Betätigungsvorrichtung erleichtern.

Um eine Befestigung des ersten Spitzenteils am Grundkörper nicht zu behindern, ist es bevorzugt, dass die Grundkörpergleitfläche und die Schlittengleitfläche zumindest abschnittsweise und im Wesentlichen miteinander fluchten, wenn sich der Grundkörper und der Schlitten in der Befestigungsstellung befinden.

Insbesondere kann der Grundkörperbefestigungsabschnitt eine Grundkörperausnehmung und der Schlittenbefestigungsabschnitt eine Schlittenausnehmung aufweisen, die in der Befestigungsstellung zumindest abschnittsweise und im Wesentlichen miteinander fluchten. Die Grundkörperausnehmung und die Schlittenausnehmung erlauben zweckmäßigerweise die, insbesondere formschlüssige, Aufnahme des ersten Befestigungsabschnitts bzw. des zweiten Befestigungsabschnitts. Durch das Fluchten dieser Ausnehmungen in der Befestigungsstellung des Schlittens zumindest im Wesentlichen lässt sich insbesondere der erste Befestigungsabschnitt durch den Schlittenbefestigungsabschnitt hindurch in den Grundkörperbefestigungsabschnitt einführen. Die Schlittenausnehmung kann dabei als Führung dienen. Anders herum lässt sich durch das Fluchten beim Lösen der Werkzeugspitze von der Betätigungsvorrichtung insbesondere der erste Befestigungsabschnitt durch den Schlitten entnehmen. Insofern können durch das Fluchten beide Spitzenteile gleichzeitig von der Betätigungsvorrichtung gelöst werden.

In einer weiteren bevorzugten Ausführungsform fluchet die Kontur des Schlittenbefestigungsabschnitts nicht mehr - insbesondere nicht mehr vollständig und im Wesentlichen - mit der Kontur des Grundkörperbefestigungsabschnitts, wenn der Schlitten aus der Befestigungsstellung heraus relativ zum Grundkörper längsverschoben ist oder wird. Dies erlaubt eine zuverlässige Sicherung des ersten Spitzenteils am Grundkörper.

Insofern ist es bevorzugt, dass der Schlittenbefestigungsabschnitt dazu eingerichtet ist, den am Grundkörperbefestigungsabschnitt befestigten ersten Spitzenteil zu sichern. Unter einem Sichern kann hierbei insbesondere ein Festlegen und/oder Arretieren des ersten Spitzenteils, insbesondere des ersten Befestigungsabschnitts, verstanden werden. Anders gesagt ist der Schlittenbefestigungsabschnitt zweckmäßigerweise dazu eingerichtet, insbesondere derart ausgebildet, dass er nach einer Längsverschiebung aus der Befestigungsstellung heraus ein Lösen des ersten Spitzenteils vom Grundkörper verhindern kann. Der Schlittenbefestigungsabschnitt kann insofern nicht nur zur Herstellung einer Verbindung zwischen dem Schlitten und dem zweiten Spitzenteil dienen, sondern auch der Befestigung des ersten Spitzenteils am Grundkörper.

Entsprechend kann es andersherum auch vorgesehen sein, dass der Schlitten dazu eingerichtet ist, den am Grundkörperbefestigungsabschnitt befestigten ersten Spitzenteil in der Befestigungsstellung freizugeben. Anders gesagt kann der Schlittenbefestigungsabschnitt so ausgestaltet sein, dass der erste Spitzenteil nur vom Grundkörper lösbar ist, wenn sich der Schlitten in der Befestigungsstellung befindet. Dadurch kann einem unbeabsichtigten Lösen der Werkzeugspitze von der Betätigungsvorrichtung entgegengewirkt werden.

In einer weiteren bevorzugten Ausführungsform ist der Schlitten im Schlittenbefestigungsabschnitt zum zumindest abschnittsweisen Umfassen des zweiten Spitzenteils gegabelt ausgebildet. Insbesondere kann der Schlitten im Schlittenbefestigungsabschnitt zum seitlichen Umfassen des zweiten Spitzenteils, insbesondere des zweiten Befestigungsabschnitts, ausgebildet sein. Unter einem seitlichen Umfassen kann hierbei ein Umfassen von (Querschnitts-)Flächen, deren Flächennormalen parallel zu Flächennormalen der ersten und zweiten Gleitfläche ausgerichtet sind, verstanden werden. Durch die gegabelte Ausbildung des Schlittens kann der zweite Befestigungsabschnitt durch eine Längsverschiebung des Schlittens relativ zum Grundkörper aus der Befestigungsstellung heraus in den Schlittenbefestigungsabschnitt, d. h. "frontal" in den Schlitten, eingeführt werden.

Zum zuverlässigen, insbesondere seitlichen, Umfassen des zweiten Spitzenteils im zweiten Befestigungsabschnitt weist der Schlittenbefestigungsabschnitt vorzugsweise zwei Gabelzinken auf. Dabei können die Gabelzinken blattartig, d. h. dünn im Hinblick auf eine Längserstreckung, ausgebildet sein. Dadurch lässt sich die Steifigkeit der Gabelzinken reduzieren.

Insofern ist es besonders bevorzugt, dass die Gabelzinken zur Befestigung des zweiten Spitzenteils durch Längsverschiebung des Schlittens verformbar ausgebildet sind. D. h., dass die Gabelzinken zum Aufschieben auf den zweiten Spitzenteil insbesondere seitlich aufbiegbar ausgebildet sein können. Dadurch kann der Schlittenbefestigungsabschnitt beispielsweise über eine, insbesondere seitliche, Ausbauchung des zweiten Befestigungsabschnitts geschoben werden und diese hintergreifen.

Insofern ist es insbesondere bevorzugt, dass der Schlittenbefestigungsabschnitt dazu eingerichtet ist, am zweiten Spitzenteil zur Befestigung einzurasten. Insofern kann der Schlittenbefestigungsabschnitt mit dem zweiten Befestigungsabschnitt einen Rastmechanismus bilden, sodass der Schlittenbefestigungsabschnitt am zweiten Befestigungsabschnitt bei Betätigung der Betätigungsvorrichtung und der daraus resultierenden Längsverschiebung des Schlittens am zweiten Spitzenteil einrastet. Dies erlaubt eine einfache und aufwandsarme Herstellung einer Verbindung zwischen dem Schlitten und dem zweiten Spitzenteil. Gleichzeitig kann durch das bei Betätigung der Betätigungsvorrichtung automatisch ausgelöste Einrasten die Sicherheit gegen ein unbeabsichtigtes lösen der Werkzeugspitze von der Betätigungsvorrichtung erhöht werden. Dabei kann auf weitere gesonderte Sicherungssysteme wie zum Beispiel Hebel oder Rasten verzichtet werden.

Ein dritter Aspekt der Erfindung betrifft ein chirurgisches Instrument. Das Instrument weist zweckmäßigerweise eine Betätigungsvorrichtung gemäß dem zweiten Aspekt der Erfindung und eine medizinischen Werkzeugspitze gemäß dem ersten Aspekt der Erfindung auf. Ein solches Instrument ist, insbesondere durch selbständiges Sichern und Einrasten beim Betätigen, einfach und sicher zu bedienen. Durch die Wiederverwendbarkeit zumindest der Betätigungsvorrichtung kann Rohmaterial, Lagerplatz und Energie eingespart werden. Insofern ist das Instrument besonders umweltfreundlich und kostengünstig.

Ein vierter Aspekt der Erfindung betrifft ein Verfahren zur Befestigung einer medizinischen Werkzeugspitze, insbesondere gemäß dem ersten Aspekt der Erfindung, an einer Betätigungsvorrichtung, insbesondere gemäß dem zweiten Aspekt der Erfindung. Die medizinische Werkzeugspitze weist zweckmäßigerweise einen ersten Spitzenteil und einen am ersten Spitzenteil längsverschiebbar, insbesondere werkzeugfrei unlösbar, befestigten zweiten Spitzenteil auf. Die Betätigungsvorrichtung weist zweckmäßigerweise einen Grundkörper und einen längsverschiebbar am Grundkörper, insbesondere werkzeugfrei unlösbar, befestigten Schlitten auf. Bei dem Verfahren wird der Schlitten durch Längsverschieben relativ zum Grundkörper in eine Befestigungsstellung gebracht und der erste Spitzenteil mithilfe eines ersten Befestigungsabschnitts am Grundkörper in einem Grundkörperbefestigungsabschnitt werkzeugfrei, insbesondere formschlüssig, befestigt. Zudem wird, insbesondere anschließend, der zweite Spitzenteils mithilfe eines zweiten Befestigungsabschnitts am Schlitten in einem Schlittenbefestigungsabschnitt durch Längsverschieben des Schlittens relativ zum Grundkörper aus der Befestigungsstellung hinaus werkzeugfrei, insbesondere formschlüssig, befestigt.

Dieses Verfahren erlaubt den schnellen Wechsel unterschiedlicher Werkzeugspitzen auch unter Stress und sterilen Bedingungen, zum Beispiel im Operationssaal. Insbesondere können scharf schneidende Werkzeugspitzen einfacher und schneller ersetzt werden, was zu kürzeren Operationszeiten führen kann.

Im Weiteren wird die Erfindung anhand von Figuren näher erläutert. Soweit zweckdienlich, sind hierin gleich wirkende Elemente mit gleichen Bezugszeichen versehen. Die Erfindung ist nicht auf die in den Figuren dargestellten Ausführungsbeispiele beschränkt - auch nicht in Bezug auf funktionale Merkmale. Die bisherige Beschreibung wie auch die nachfolgende Figurenbeschreibung enthalten zahlreiche Merkmale, die in den abhängigen Unteransprüchen teilweise zu mehreren zusammengefasst wiedergegeben sind. Diese Merkmale wie auch alle übrigen oben und in der nachfolgenden Figurenbeschreibung offenbarten Merkmale wird der Fachmann jedoch auch einzeln betrachten und zu sinnvollen weiteren Kombinationen zusammenfügen. Insbesondere sind alle genannten Merkmale jeweils einzeln und in beliebiger geeigneter Kombination mit der medizinischen Werkzeugspitze gemäß dem ersten Aspekt der Erfindung, der Betätigungsvorrichtung gemäß dem zweiten Aspekt der Erfindung, dem chirurgischen Instrument gemäß dem dritten Aspekt der Erfindung und dem Verfahren gemäß dem vierten Aspekt der Erfindung kombinierbar.

Es zeigen, insbesondere zumindest teilweise schematisch:
- Fig. 1: ein Beispiel eines chirurgischen Instruments;
- Fig. 2: Baugruppen des chirurgischen Instruments aus Figur 1;
- Fig. 3: ein Beispiel einer medizinischen Werkzeugspitze in einer Seitenansicht;
- Fig. 4: die medizinische Werkzeugspitze aus Figur 3 in zerlegter Form;
- Fig. 5: die medizinische Werkzeugspitze aus Fig. 3 in einer Draufsicht;
- Fig. 6: ein Beispiel eines distalen Endes einer Betätigungsvorrichtung in einer Schnittansicht;
- Fig. 7: das distale Ende der Betätigungsvorrichtung aus Figur 6 in einer dreidimensionalen Darstellung; und
- Fig. 8: ein Beispiel eines Verfahrens zum Befestigen einer medizinischen Werkzeugspitze an einer Betätigungsvorrichtung.

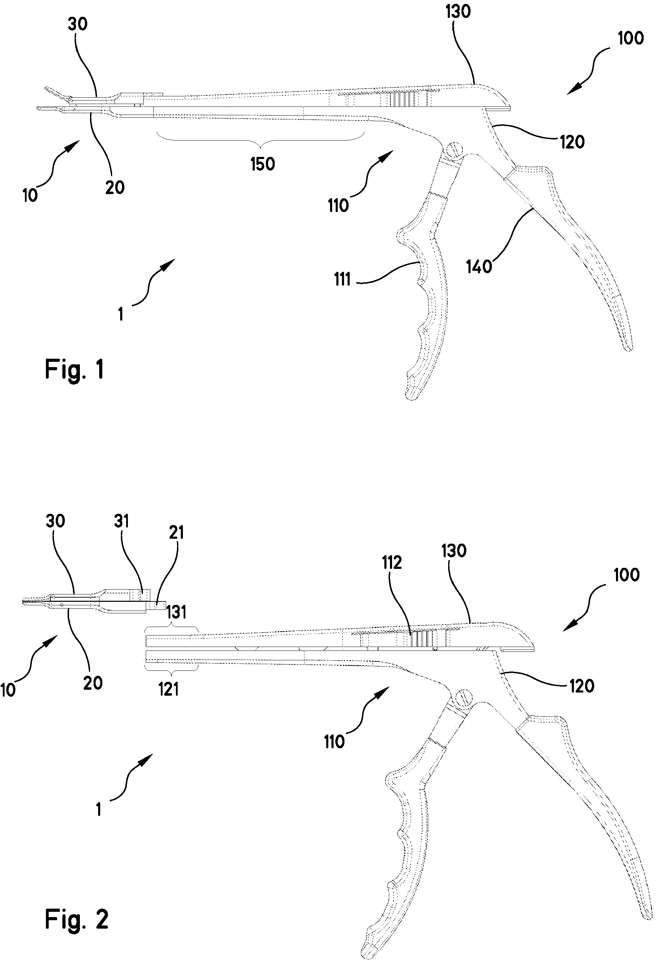

Figur 1 zeigt ein Beispiel eines chirurgischen Instruments 1 mit einer medizinischen Werkzeugspitze 10 und einer Betätigungsvorrichtung 100. Die Werkzeugspitze 10 weist einen ersten Spitzenteil 20 und einen am ersten Spitzenteil 20 zumindest abschnittsweise längsverschiebbar befestigten zweiten Spitzenteil 30 auf. Die Betätigungsvorrichtung 100 weist einen Grundkörper 120 und einen am Grundkörper 120 zumindest abschnittsweise längsverschiebbar befestigten Schlitten 130 auf. Die Längsverschiebung des Schlittens 130 relativ zum Grundkörper 120 kann beispielsweise mithilfe eines Betätigungsmechanismus 110 ausgelöst werden.

An einem proximalen, d. h. einem Benutzer bei Benutzung des Instruments 1 zugewandten, Ende weist die Betätigungsvorrichtung 100 vorzugsweise einen Griff 140 auf. Es ist zweckmäßig, dass der Griff 140 vom Grundkörper 120 gebildet ist. Ein anderer Teil des Grundkörpers 120 bildet, zusammen mit einem entsprechenden Abschnitt des Schlittens 130, einen Schaft 150.

An einem distalen, d. h. einem vom Benutzer bei Benutzung des chirurgischen Instruments 1 abgewandten, Ende der Betätigungsvorrichtung 100, insbesondere des Schafts 150, ist die Werkzeugspitze 10 angeordnet. Die Werkzeugspitze 10 ist eine auswechselbare Werkzeugspitze (siehe Figur 2) und in der vorliegenden Darstellung betriebsbereit mit der Betätigungsvorrichtung 100 verbunden. Dazu ist zweckmäßigerweise der erste Spitzenteil 20 am Grundkörper 120 und der zweite Spitzenteil 30 am Schlitten 130 befestigt. Zum Beispiel aufgrund einer kürzeren Ausgestaltung des zweiten Spitzenteils 30 gegenüber dem ersten Spitzenteil 20 ragt der Schlitten 130 dabei am distalen Ende der Betätigungsvorrichtung 100 über den Grundkörper 120 hinaus.

Bei Auslösen des Betätigungsmechanismus 110, insbesondere durch Ziehen eines Auslösehebels 111, verschiebt sich der Schlitten 130 relativ zum Grundkörper 120 zum distalen Ende hin. Durch die Verbindung des Schlittens 130 mit dem zweiten Spitzenteil 30 kann so die Werkzeugspitze 10 betätigt werden.

Bei der in Figur 1 gezeigten Werkzeugspitze 10 handelt es sich rein beispielhaft um eine sogenannte Löffel- oder Fasszange. Das chirurgische Instrument 1 ist jedoch auch mit einer anderen Werkzeugspitze denkbar, zum Beispiel mit einer Stanze, einem Bandscheibenaufsatz, einem Nadelhalter, einer Klemme oder einer Schere.

Figur 2 zeigt Baugruppen des chirurgischen Instruments 1 aus Figur 1, nämlich insbesondere die Betätigungsvorrichtung 100 und die davon gelöste Werkzeugspitze 10. Dadurch ist ein erster Befestigungsabschnitt 21 des ersten Spitzenteils 20 und ein zweiter Befestigungsabschnitt 31 des zweiten Spitzenteils 30 sichtbar. Der Schlitten 130 befindet sich relativ zum Grundkörper 120 in einer Befestigungsstellung, in welcher insbesondere der erste Spitzenteil 20 am Grundkörper 120 befestigbar (oder lösbar) ist.

Die Befestigungsabschnitte 21, 31 dienen zweckmäßigerweise der Befestigung des ersten und zweiten Spitzenteils 20, 30 am Grundkörper 120 bzw. Schlitten 130. Der erste Befestigungsabschnitt 21 ist zur Befestigung am Grundkörper 120 dabei in bevorzugter Weise in einen Grundkörperbefestigungsabschnitt 121 am distalen Ende des Grundkörpers einführbar. Anders gesagt ist der Grundkörper 120 im Grundkörperbefestigungsabschnitt 121 zweckmäßigerweise dazu eingerichtet, den ersten Befestigungsabschnitt 21 insbesondere formschlüssig aufzunehmen.

Es ist zweckdienlich, dass der Grundkörperbefestigungsabschnitt 121 dabei nur in der Befestigungsstellung des Schlittens 130 für den ersten Befestigungsabschnitt 21 zugänglich ist. Insofern ist es bevorzugt, dass der Schlitten 130 den ersten Befestigungsabschnitt 21 im Grundkörperbefestigungsabschnitt 121 in zumindest einer Betriebsstellung sichert. Eine solche Betriebsstellung liegt zweckmäßigerweise vor, wenn der Schlitten 130 - wie im in Figur 1 gezeigten betriebsbereiten Zustand des chirurgischen Instruments 1 - am distalen Ende über den Grundkörper 120, insbesondere den Grundkörperbefestigungsabschnitt 121, hinausragt.

Der zweite Befestigungsabschnitt 31 ist zur Befestigung des zweiten Spitzenteils 30 am Schlitten 130 in bevorzugter Weise in einen Schlittenbefestigungsabschnitt 131 am distalen Ende des Schlittens 130 einführbar. Anders gesagt ist der Schlitten 130 im Schlittenbefestigungsabschnitt 131 zweckmäßigerweise dazu eingerichtet, den zweiten Befestigungsabschnitt 31 insbesondere formschlüssig aufzunehmen. Dabei kann der Schlittenbefestigungsabschnitt 131 zum Aufschieben auf den, insbesondere zum Einrasten am, zweiten Befestigungsabschnitt 31 eingerichtet sein. Eine Verbindung zwischen dem Schlitten 130 und dem zweiten Spitzenteil 30 ist dabei vorzugsweise einfach durch Längsverschiebung des Schlittens 130 relativ zum Grundkörper 120, d. h. durch Betätigung des Betätigungsmechanismus 110, herstellbar.

Zweckmäßigerweise ist der Schlitten 130 in der Befestigungsstellung - wie in Figur 2 dargestellt - vom Grundkörper 120 abgehoben. Dies kann nicht nur das Ablösen und Befestigen des ersten Spitzenteils 20 vom bzw. am Grundkörper 120 erleichtern, sondern auch eine Reinigung bzw. Sterilisation der Betätigungsvorrichtung 100. Daher kann die Befestigungsstellung auch als Reinigungs- oder Sterilisationsstellung bezeichnet werden. Die Betätigungsvorrichtung 100 kann aus dem in Figur 1 gezeigten betriebsbereiten Zustand in die Befestigungsstellung gebracht werden, indem beispielsweise eine Arretierung des Betätigungsmechanismus 110 gelöst und der Schlitten 130 relativ zum Grundkörper 120 händisch zum proximalen Ende hin gezogen wird. Zur Lösung der Arretierung ist zweckmäßigerweise ein entsprechendes Betätigungselement 112 am Schlitten 130 vorgesehen.

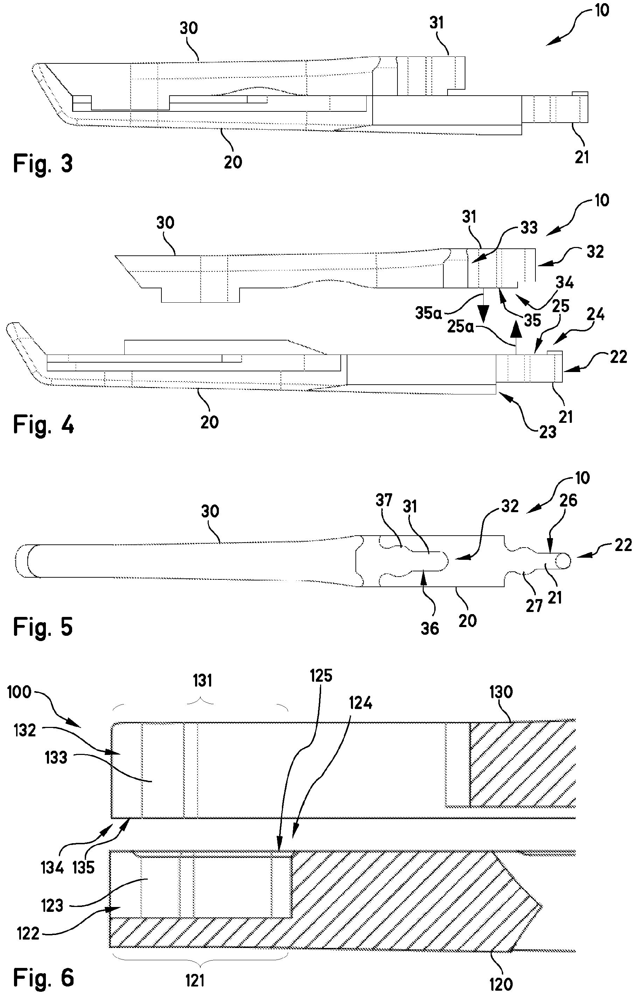

Figur 3 zeigt ein Beispiel einer medizinischen Werkzeugspitze 10 in einer Seitenansicht. Die Werkzeugspitze 10 weist einen ersten Spitzenteil 20 und einen am ersten Spitzenteil 20 längsverschiebbar befestigten zweiten Spitzenteil 30 auf. Der erste Spitzenteil 20 weist dabei einen ersten Befestigungsabschnitt 21 und der zweite Spitzenteil 30 einen zweiten Befestigungsabschnitt 31 auf, die jeweils zur Befestigung an einem Grundkörper bzw. Schlitten einer Betätigungsvorrichtung (siehe Figuren 1 und 2) eingerichtet sind.

Zweckmäßigerweise sind der erste Befestigungsabschnitt 21 und der zweite Befestigungsabschnitt 31 derart ausgebildet, insbesondere angeordnet, dass erst der erste Befestigungsabschnitt 21 am Grundkörper und anschließend der zweite Befestigungsabschnitt 31 am Schlitten befestigbar ist. Anders gesagt können der erste und zweite Befestigungsabschnitt 21, 31 derart ausgebildet, insbesondere angeordnet, sein, dass eine zweistufige Befestigung der Werkzeugspitze 10 an der Betätigungsvorrichtung möglich ist. Beispielsweise kann zunächst der erste Befestigungsabschnitt 21 in einen Grundkörperbefestigungsabschnitt des Grundkörpers eingehängt werden, woraufhin ein Schlittenbefestigungsabschnitt des Schlittens durch Längsverschiebung relativ zum Grundkörper auf den zweiten Befestigungsabschnitt 31 aufschiebbar ist. Insbesondere kann durch das Befestigen des ersten Befestigungsabschnitts 21 am Grundkörper der zweite Befestigungsabschnitt 31 derart positioniert und/oder gehalten sein, um das Herstellen einer Verbindung zwischen dem zweiten Befestigungsabschnitt 31 und dem Schlitten zu ermöglichen oder zumindest zu vereinfachen.

Zur zweistufigen Befestigung an der Betätigungsvorrichtung können die Befestigungsabschnitte 21, 31 beispielsweise, insbesondere in einer Längsrichtung der Werkzeugspitze 10, voneinander beabstandet sein. Auf einfache Weise lässt sich eine solche Anordnung erreichen, indem der zweite Spitzenteil 30 kürzer ausgebildet ist als der erste Spitzenteil 20.

Figur 4 zeigt die Werkzeugspitze 10 aus Figur 3 in zerlegter Form. Hierbei ist eine mögliche Ausgestaltung des ersten und zweiten Befestigungsabschnitts 21, 31 dargestellt. Der erste Befestigungsabschnitt 21 und der zweite Befestigungsabschnitt 31 können hierbei jeweils einen ersten bzw. zweiten Vorsprung 22, 32 aufweisen, der sich längsverlaufend an ein proximales Ende 23, 33 des jeweiligen Spitzenteils 20, 30 anschließt. Insbesondere können der erste und zweite Befestigungsabschnitt 21, 31 durch den jeweiligen Vorsprung 22, 32 definiert sein. Die Vorsprünge 22, 32 sind zweckmäßigerweise derart ausgeformt, dass sie insbesondere formschlüssig und festsitzend vom Grundkörper im Grundkörperbefestigungsabschnitt bzw. vom Schlitten im Schlittenbefestigungsabschnitt aufnehmbar sind.

Um eine reibungslose Betätigung der Werkzeugspitze 10 zu ermöglichen, ist es zweckdienlich, dass der erste Spitzenteil 20, insbesondere der erste Befestigungsabschnitt 21, eine Oberseite 24 mit einer ebenen Oberseitenfläche 25 aufweist. Die Oberseite 24 ist zweckmäßigerweise einer Unterseite 34 des zweiten Spitzenteils 30, insbesondere des zweiten Befestigungsabschnitts 31, zugewandt. Die Unterseite 34 weist zweckmäßigerweise eine ebene Unterseitenfläche 35 auf.

Es ist bevorzugt, dass die Oberseitenfläche 25 und die Unterseitenfläche 35 zweckmäßigerweise parallel zueinander verlaufen. In Figur 4 sind die entsprechenden Flächennormalen 25a, 35a der Oberseitenfläche 25 bzw. der Unterseitenfläche 35 eingezeichnet.

Figur 5 zeigt die Werkzeugspitze 10 aus Figur 3 in einer Draufsicht. Hierbei ist eine mögliche Ausformung des ersten Spitzenteils 20 im ersten Befestigungsabschnitt 21, insbesondere des ersten Vorsprungs 22, und des zweiten Spitzenteils 30 im zweiten Befestigungsabschnitt 31, insbesondere des zweiten Vorsprungs 32, dargestellt.

Der erste und zweite Vorsprung 22, 32 weisen zweckmäßigerweise Seitenwände 26, 36 auf. Diese Seitenwände 26, 36 können senkrecht zu der in Figur 4 eingezeichneten Oberseitenfläche bzw. Unterseitenfläche, d. h. senkrecht zur Figurenebene in Figur 5, verlaufen. Zur zuverlässigen, insbesondere formschlüssigen, Befestigung an der Betätigungsvorrichtung sind die Seitenwände 26, 36 in bevorzugter Weise uneben ausgebildet.

Beispielsweise können der erste und zweite Befestigungsabschnitt 21, 31 jeweils eine erste bzw. zweite Ausbauchung 27, 37 aufweisen. Diese Ausbauchungen 27, 37 verlaufen zweckmäßigerweise quer zu den in Figur 4 eingezeichneten Flächennormalen der Oberseitenfläche bzw. Unterseitenfläche. Anders gesagt weisen die Vorsprünge 22, 32 vorzugsweise eine seitliche - d. h. im Bereich der Seitenwände 26, 36 angeordnete - Verdickung auf.

Die Ausbauchungen 27, 37 weisen vorzugsweise jeweils eine konvexe Krümmung auf. Der erste und der zweite Befestigungsabschnitt 21, 31 können dadurch derart ausgeformt sein, dass der erste bzw. zweite Befestigungsabschnitt 21, 31 vom Grundkörper bzw. vom Schlitten hintergriffen werden kann. Dadurch kann eine ungewollte Ablösung der Werkzeugspitze 10 von dem distalen Ende der Betätigungsvorrichtung in Längsrichtung verhindert werden.

Wie in Figur 5 gut zu erkennen ist, weisen der erste Befestigungsabschnitt 21 und der zweite Befestigungsabschnitt 31 in einer möglichen Ausgestaltung zumindest abschnittsweise und zumindest im Wesentlichen die gleiche Kontur, insbesondere die gleiche Außenkontur, auf. Insbesondere können die in Figur 4 eingezeichnete Oberseitenfläche 25 und die dort ebenfalls eingezeichnete Unterseitenfläche 35 zumindest abschnittsweise gleichartig ausgeformt sein. Insofern ist es zweckdienlich, wenn die Seitenwände 26, 36 gleichartig uneben ausgebildet sind, d. h. zum Beispiel die zweite Ausbauchung 37 zumindest im Wesentlichen gleich der ersten Ausbauchung 27 ausgebildet ist.

Dabei ist es nicht zwingend, dass die Konturen der Befestigungsabschnitte 21, 31 identisch sind, d. h. sich ohne Skalierung aufeinander abbilden lassen. Vielmehr kann insbesondere der erste Befestigungsabschnitt 21 kleiner bemaßt, insbesondere schmaler ausgebildet, sein als der zweite Befestigungsabschnitt 31. Es ist jedoch bevorzugt, dass sich die Konturen gleichen, d. h. sich bei entsprechender geringfügiger Skalierung zumindest abschnittsweise aufeinander abbilden lassen.

Ein derartiges Korrespondieren des ersten Befestigungsabschnitts 21 mit dem zweiten Befestigungsabschnitt 31 erlaubt bei einer zweistufigen Befestigung der Werkzeugspitze 10 an der Betätigungsvorrichtung eine Sicherung des ersten Befestigungsabschnitts 21 im Grundkörperbefestigungsabschnitt und Aufschieben des Schlittenbefestigungsabschnitts auf den zweiten Befestigungsabschnitt 31 durch eine einzige Betätigung der Betätigungsvorrichtung. Eine kleinere Bemaßung des ersten Befestigungsabschnitts 21 kann dabei sicherstellen, dass dieser leicht durch den Schlittenbefestigungsabschnitt führbar ist. Dies wird im Zusammenhang mit Figur 6 deutlich.

Figur 6 zeigt ein Beispiel eines distalen Endes einer Betätigungsvorrichtung 100 in einer Schnittansicht. Die Betätigungsvorrichtung 100 weist einen Grundkörper 120 und einen längsverschiebbar am Grundkörper 120 befestigten Schlitten 130 auf.

Der Schlitten 130 befindet sich dabei in der bereits in Figur 2 gezeigten Befestigungsstellung, in welcher er parallel zum Grundkörper 120 verlaufend von diesem abgehoben ist. Dadurch ist in Figur 6 eine Grundkörperoberseite 124 mit einer Grundkörpergleitfläche 125 des Grundkörpers 120 sichtbar, die einer Schlittenunterseite 134 des Schlittens 130 mit einer Schlittengleitfläche 135 zugewandt ist. Die Schlittengleitfläche 135 ist zweckmäßigerweise dazu eingerichtet, bei Betätigung der Betätigungsvorrichtung 100 an der Grundkörpergleitfläche 125 entlang zu gleiten.

Der Grundkörper 120 weist einen Grundkörperbefestigungsabschnitt 121 zur Befestigung eines ersten Spitzenteils einer medizinischen Werkzeugspitze (siehe Figuren 3, 4 und 5) auf. Der Grundkörperbefestigungsabschnitt 121 kann insbesondere dazu eingerichtet sein, einen ersten Befestigungsabschnitt des ersten Spitzenteils aufzunehmen. Dazu ist es zweckdienlich, dass der Grundkörperbefestigungsabschnitt 121 korrespondierend mit dem ersten Befestigungsabschnitt ausgebildet, insbesondere ausgeformt, ist. Insbesondere kann der Grundkörperbefestigungsabschnitt 121 eine Grundkörperausnehmung 122 aufweisen, in die ein erster Vorsprung des ersten Spitzenteils (siehe Figuren 4 und 5) formschlüssig einführbar ist, d. h. die korrespondierend zum ersten Vorsprung ausgebildet ist.

Der Schlitten 130 weist einen Schlittenbefestigungsabschnitt 131 zur Befestigung eines zweiten Spitzenteils der Werkzeugspitze (siehe Figuren 3, 4 und 5) auf. Der Schlittenbefestigungsabschnitt 131 kann insbesondere dazu eingerichtet sein, einen zweiten Befestigungsabschnitt des zweiten Spitzenteils aufzunehmen. Dazu ist es zweckdienlich, dass der Schlittenbefestigungsabschnitt 131 korrespondierend mit dem zweiten Befestigungsabschnitt ausgebildet, insbesondere ausgeformt, ist. Insbesondere kann der Schlittenbefestigungsabschnitt 131 eine Schlittenausnehmung 132 aufweisen, in die ein zweiter Vorsprung des zweiten Spitzenteils (siehe Figuren 4 und 5) formschlüssig einführbar ist, d. h. die korrespondierend zum zweiten Vorsprung ausgebildet ist.

Beispielsweise können sowohl der Grundkörperbefestigungsabschnitt 121 als auch der Schlittenbefestigungsabschnitt 131 eine Einbuchtung 123, 133 aufweisen. Diese Einbuchtungen 123, 133 können dazu eingerichtet sein, entsprechende Ausbauchungen des ersten und zweiten Befestigungsabschnitts (siehe Figur 5) aufzunehmen.

Wie in Figur 6 gut zu erkennen ist, weisen der Grundkörperbefestigungsabschnitt 121 und der Schlittenbefestigungsabschnitt 131 zumindest abschnittsweise im Wesentlichen die gleiche Kontur, insbesondere die gleiche Innenkontur, auf. Beispielsweise können die Grundkörpergleitfläche 125 und die Schlittengleitfläche 135 zumindest abschnittsweise die gleiche Kontur aufweisen.

Es ist insbesondere zweckdienlich, dass die Konturen des Grundkörperbefestigungsabschnitts 121 und des Schlittenbefestigungsabschnitts 131 - wie in Figur 6 dargestellt - zumindest abschnittsweise und im Wesentlichen miteinander fluchten, wenn sich der Schlitten 130 in der Befestigungsstellung befindet. Insbesondere können dabei die Grundkörpergleitfläche 125 und die Schlittengleitfläche 135 zumindest abschnittsweise miteinander fluchten. Dadurch lässt sich ein korrespondierend zum Grundkörperbefestigungsabschnitt 121 ausgeformter erster Befestigungsabschnitt problemlos durch den Schlittenbefestigungsabschnitt 131 hindurch in den Grundkörperbefestigungsabschnitt 121 einführen.

Wird der Schlitten 130 aus der Befestigungsstellung heraus relativ zum Grundkörper 120 längsverschoben, kann der Schlitten 130 den vom Grundkörperbefestigungsabschnitt 121 aufgenommenen ersten Befestigungsabschnitt sichern. Anders gesagt kann der Schlitten 130 in einer solchen Betriebsstellung verhindern, dass der erste Befestigungsabschnitt durch den Schlittenbefestigungsabschnitt 131 hindurch (ungewollt) aus dem Grundkörperbefestigungsabschnitt 121 herausgezogen wird. Dies lässt sich beispielsweise dadurch erreichen, dass ein Teil des Schlittens 130 die Einbuchtung 123 im Grundkörperbefestigungsabschnitt 121 zumindest abschnittsweise überdeckt.

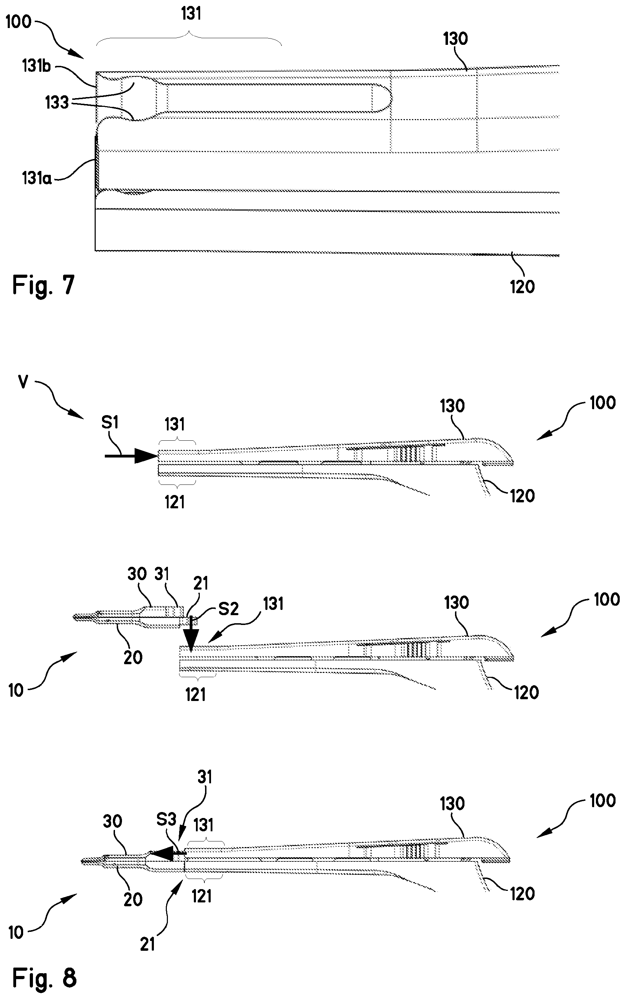

Die Befestigung des zweiten Spitzenteils am Schlitten 130 wird im Zusammenhang mit Figur 7 erläutert.

Figur 7 zeigt das distale Ende der Betätigungsvorrichtung 100 aus Figur 6 in einer dreidimensionalen Darstellung. Dabei ist gut erkennbar, dass der Schlitten 130 im Schlittenbefestigungsabschnitt 131 zweckmäßigerweise gegabelt ausgebildet ist. Der Schlitten 130 kann beispielsweise zwei Gabelzinken 131a, 131b aufweisen. Die Gabelzinken 131a, 131b sind vorzugsweise blattartig ausgebildet, d. h. dünn relativ zu einer Längserstreckung. Dadurch kann eine Flexibilität des Schlittenbefestigungsabschnitts 131 erreicht werden, die es erlaubt, den Schlittenbefestigungsabschnitt 131 durch eine Längsverschiebung auf den zweckmäßigerweise korrespondierend zum Schlittenbefestigungsabschnitt 131 ausgeformten zweiten Befestigungsabschnitt zu schieben.

Beispielsweise können die Gabelzinken 131a, 131b bei einer Längsbewegung zum zweiten Spitzenteil hin durch die Ausbauchung im zweiten Befestigungsabschnitt (siehe Figur 5) seitlich auseinandergedrückt bzw. aufgebogen werden. Erreicht die Ausbauchung schließlich die Einbuchtungen 133, kann der Schlittenbefestigungsabschnitt 131 am zweiten Spitzenteil einrasten, indem die Ausbauchung hintergriffen wird.

Figur 8 zeigt ein Beispiel eines Verfahrens V zum Befestigen einer medizinischen Werkzeugspitze 10 an einer Betätigungsvorrichtung 100.

In einem Verfahrensschritt S1 wird ein Schlitten 130 der Betätigungsvorrichtung 100 durch Längsverschieben relativ zu einem Grundkörper 120 der Betätigungsvorrichtung 100 in eine Befestigungsstellung gebracht. Dabei kann der Schlitten 130 insbesondere derart längsverschoben werden, dass eine Kontur eines Schlittenbefestigungsabschnitts 131 mit einer Kontur eines Grundkörperbefestigungsabschnitts 121 fluchtet. Beispielsweise kann eine Schlittenausnehmung des Schlittenbefestigungsabschnitts 131 fluchtend mit einer Grundkörperausnehmung des Grundkörperbefestigungsabschnitts 121 ausgerichtet werden.

In einem weiteren Verfahrensschritt S2 wird ein erster Befestigungsabschnitt 21 eines ersten Spitzenteils 20 der Werkzeugspitze 10 im Grundkörperbefestigungsabschnitt 121 des Grundkörpers 120 befestigt. Insbesondere kann der erste Befestigungsabschnitt 21 von oberhalb der Betätigungsvorrichtung 100, d. h. von einer dem Grundkörper 120 abgewandten Oberseite des Schlittens 130 aus, wie durch den Pfeil angedeutet in den Grundkörperbefestigungsabschnitt 121, insbesondere die Grundkörperausnehmung, eingesetzt werden. Dabei wird der erste Befestigungsabschnitt 21 zweckmäßigerweise durch den Schlittenbefestigungsabschnitt 131 hindurch geführt.

In einem weiteren Verfahrensschritt S3 wird ein zweiter Befestigungsabschnitt 31 eines zweiten Spitzenteils 30 der Werkzeugspitze 10 durch Längsverschieben des Schlittens 130 relativ zum Grundkörper 120 aus der Befestigungsstellung heraus befestigt. Zweckmäßigerweise wird der erste Befestigungsabschnitt 21 dabei gleichzeitig im Grundkörperbefestigungsabschnitt 121 gesichert, zum Beispiel indem der Schlitten 130 den in einer Betätigungsstellung nach oben offenen Grundkörperbefestigungsabschnitt 121 zumindest teilweise abdeckt.

Zweckmäßigerweise schiebt sich der Schlittenbefestigungsabschnitt 131 dabei in einer Längsrichtung wie durch den Pfeil angedeutet auf den zweiten Befestigungsabschnitt 31, bis der Schlitten 130 am zweiten Befestigungsabschnitt 31 einrastet. Der zweite Befestigungsabschnitt 31 wird dabei in bevorzugter Weise seitlich umfasst.

Es ist infolgedessen möglich, durch eine einfache Betätigung der Betätigungsvorrichtung 100 eine Verbindung zwischen dem Schlitten 130 und dem zweiten Spitzenteil 30 herzustellen und gleichzeitig den ersten Spitzenteil 20 am Grundkörper 120 festzulegen. Dies kann eine signifikante Erhöhung der Benutzerfreundlichkeit bedeuten und einen sicheren Betrieb des derart zusammengesetzten chirurgischen Instruments erlauben.

### Bezugszeichenliste

- 1: chirurgisches Instrument
- 10: medizinische Werkzeugspitze
- 20: erster Spitzenteil
- 21: erster Befestigungsabschnitt
- 22: erster Vorsprung
- 23: proximales Ende des ersten Spitzenteils
- 24: Oberseite
- 25: Oberseitenfläche
- 25a: Flächennormale der Oberseitenfläche
- 26: Seitenwand
- 27: erste Ausbauchung
- 30: zweiter Spitzenteil
- 31: zweiter Befestigungsabschnitt
- 32: zweiter Vorsprung
- 33: proximales Ende des zweiten Spitzenteils
- 34: Unterseite
- 35: Unterseitenfläche
- 35a: Flächennormale der Unterseitenfläche
- 36: Seitenwand
- 37: zweite Ausbauchung
- 100: Betätigungsvorrichtung
- 110: Betätigungsmechanismus
- 111: Auslösehebel
- 112: Betätigungselement
- 120: Grundkörper
- 121: Grundkörperbefestigungsabschnitt
- 122: Grundkörperausnehmung
- 123: Einbuchtung
- 124: Grundkörperoberseite
- 125: Grundkörpergleitfläche
- 130: Schlitten
- 131: Schlittenbefestigungsabschnitt
- 131a: Gabelzinken
- 131b: Gabelzinken
- 132: Schlittenausnehmung
- 133: Einbuchtung
- 134: Schlittenunterseite
- 135: Schlittengleitfläche
- 140: Griff
- 150: Schaft
- V: Verfahren
- S1-S3: Verfahrensschritte

## Patentansprüche

1. Medizinische Werkzeugspitze (10) für ein chirurgisches Instrument (1), aufweisend
- einen ersten Spitzenteil (20) mit einem ersten Befestigungsabschnitt (21) zur werkzeugfreien Befestigung des ersten Spitzenteils (20) an einem Grundkörper (120) einer Betätigungsvorrichtung (100) und
- einen zweiten Spitzenteil (30) mit einem zweiten Befestigungsabschnitt (31) zur werkzeugfreien Befestigung des zweiten Spitzenteils (30) an einem Schlitten (130) der Betätigungsvorrichtung (100), wobei der zweite Spitzenteil (30) zumindest abschnittsweise längsverschiebbar am ersten Spitzenteil (20) befestigt ist,
**dadurch gekennzeichnet,**
**dass** der erste und zweite Befestigungsabschnitt (21, 31) derart ausgebildet sind, dass eine Befestigung des zweiten Spitzenteils (30) am Schlitten (130) durch zumindest abschnittsweises Längsverschieben des Schlittens (130) erst erfolgen kann, wenn der erste Spitzenteil (20) bereits am Grundkörper (120) befestigt und gesichert ist.

2. Werkzeugspitze (10) nach Anspruch 1,
**dadurch gekennzeichnet,**
**dass** der erste Befestigungsabschnitt (21) und der zweite Befestigungsabschnitt (31) zumindest abschnittsweise die gleiche Kontur aufweisen.

3. Werkzeugspitze (10) nach einem der vorangehenden Ansprüche,
**dadurch gekennzeichnet,**
**dass**
- der erste Befestigungsabschnitt (21) einen ersten Vorsprung (22) aufweist, der sich längsverlaufend an ein proximales Ende (23) des ersten Spitzenteils (20) anschließt und eine Oberseite (24) mit einer ebenen Oberseitenfläche (25) aufweist,
- der zweite Befestigungsabschnitt (31) einen zweiten Vorsprung (32) aufweist, der sich längsverlaufend an ein proximales Ende (33) des zweiten Spitzenteils (30) anschließt und eine Unterseite (34) mit einer ebenen Unterseitenfläche (35) aufweist, die im Wesentlichen parallel zur Oberseitenfläche (25) ausgerichtet ist, und
- der erste und zweite Vorsprung (22, 32) jeweils Seitenwände (26, 36) aufweisen, die im Wesentlichen senkrecht zur Oberseitenfläche (25) und zur Unterseitenfläche (35) verlaufen und gleichartig uneben ausgebildet sind.

4. Werkzeugspitze (10) nach einem der vorangehenden Ansprüche,
**dadurch gekennzeichnet,**
**dass**
- der erste Spitzenteil (20) im ersten Befestigungsabschnitt (21) eine Oberseite (24) mit einer Oberseitenfläche (25) und eine erste Ausbauchung (27) aufweist, die quer zu einer Flächennormalen (25a) der Oberseitenfläche (25) verläuft, und
- der zweite Spitzenteil (30) im zweiten Befestigungsabschnitt (31) eine Unterseite (34) mit einer der Oberseitenfläche (25) zugewandte Unterseitenfläche (35) und eine zweite Ausbauchung (37) aufweist, die quer zu einer Flächennormalen (35a) der Unterseitenfläche (35) verläuft und gleichartig zur ersten Ausbauchung (27) ausgeformt ist.

5. Betätigungsvorrichtung (100) für ein chirurgisches Instrument (1), aufweisend
- einen Grundkörper (120) mit einem Grundkörperbefestigungsabschnitt (121) zur werkzeugfreien Befestigung eines ersten Spitzenteils (20) einer medizinischen Werkzeugspitze (10) am Grundkörper (120) und
- einen Schlitten (130) mit einem Schlittenbefestigungsabschnitt (131) zur werkzeugfreien Befestigung eines am ersten Spitzenteil (20) längsverschiebbar befestigten zweiten Spitzenteils (30) der Werkzeugspitze (10) am Schlitten (130), wobei der Schlitten (130) zumindest abschnittsweise längsverschiebbar am Grundkörper (120) befestigt ist,
**dadurch gekennzeichnet,**
**dass** zur werkzeugfreien Befestigung der Werkzeugspitze (10) an der Betätigungsvorrichtung (100) der Schlitten (130)
- durch zumindest abschnittsweises Längsverschieben relativ zum Grundkörper (120) in eine Befestigungsstellung bringbar ist, in welcher der erste Spitzenteil (20) im Grundkörperbefestigungsabschnitt (121) am Grundkörper (120) befestigbar ist, und
- derart ausgebildet ist, dass ein Einführen eines ersten Befestigungsabschnitts (21) des ersten Spitzenteils (20) in den Grundkörperbefestigungsabschnitt (121) lediglich in der Befestigungsstellung möglich ist und der zweite Spitzenteil (30) bei am Grundkörper (120) befestigten ersten Spitzenteil (20) durch zumindest abschnittsweises Längsverschieben des Schlittens (130) aus der Befestigungsstellung heraus im Schlittenbefestigungsabschnitt (131) mit dem Schlitten (130) verbindbar ist.

6. Betätigungsvorrichtung (100) nach Anspruch 5,
**dadurch gekennzeichnet,**
**dass** eine Kontur des Grundkörperbefestigungsabschnitts (121) und eine Kontur des Schlittenbefestigungsabschnitts (131) zumindest abschnittsweise und im Wesentlichen miteinander fluchten, wenn sich der Schlitten (130) in der Befestigungsstellung befindet.

7. Betätigungsvorrichtung (100) nach einem der Ansprüche 5 oder 6,
**dadurch gekennzeichnet,**
**dass**
- der Grundkörper (120) eine dem Schlitten (130) zugewandte Grundkörperoberseite (124) mit einer ebenen Grundkörpergleitfläche (125) aufweist,
- der Schlitten (130) eine dem Grundkörper (120) zugewandte Schlittenunterseite (134) mit einer ebenen Schlittengleitfläche (135) aufweist, mit welcher der Schlitten (130) bei einer Längsverschiebung relativ zum Grundkörper (120) zumindest abschnittsweise an der Grundkörpergleitfläche (125) entlanggleitet, und
- die Grundkörpergleitfläche (125) und die Schlittengleitfläche (135) zumindest abschnittsweise und im Wesentlichen miteinander fluchten, wenn sich der Schlitten (130) in der Befestigungsstellung befindet.

8. Betätigungsvorrichtung (100) nach einem der Ansprüche 5 bis 7,
**dadurch gekennzeichnet,**
**dass** der Grundkörperbefestigungsabschnitt (121) eine Grundkörperausnehmung (122) und der Schlittenbefestigungsabschnitt (131) eine Schlittenausnehmung (132) aufweist, die in der Befestigungsstellung zumindest abschnittsweise und im Wesentlichen miteinander fluchten.

9. Betätigungsvorrichtung (100) nach einem der Ansprüche 5 bis 8,
**dadurch gekennzeichnet,**
**dass** der Schlittenbefestigungsabschnitt (131) dazu eingerichtet ist, den am Grundkörperbefestigungsabschnitt (121) befestigten ersten Spitzenteil (20) zu sichern.

10. Betätigungsvorrichtung (100) nach einem der Ansprüche 5 bis 9,
**dadurch gekennzeichnet,**
**dass** der Schlitten (130) dazu eingerichtet ist, den am Grundkörperbefestigungsabschnitt (121) befestigten ersten Spitzenteil (20) in der Befestigungsstellung freizugeben.

11. Betätigungsvorrichtung (100) nach einem der Ansprüche 5 bis 10,
**dadurch gekennzeichnet,**
**dass** der Schlitten (130) im Schlittenbefestigungsabschnitt (131) zum zumindest abschnittsweisen Umfassen des zweiten Spitzenteils (30) gegabelt ausgebildet ist.

12. Betätigungsvorrichtung (100) nach einem der Ansprüche 5 bis 11,
**dadurch gekennzeichnet,**
**dass** der Schlittenbefestigungsabschnitt (131) zwei Gabelzinken (131a, 131b) aufweist, die zur Befestigung des zweiten Spitzenteils (30) durch Längsverschiebung des Schlittens (130) verformbar ausgebildet sind.

13. Betätigungsvorrichtung (100) nach einem der Ansprüche 5 bis 12,
**dadurch gekennzeichnet,**
**dass** der Schlittenbefestigungsabschnitt (131) dazu eingerichtet ist, am zweiten Spitzenteil (30) zur Befestigung einzurasten.

14. Chirurgisches Instrument (1) mit einer Betätigungsvorrichtung (100) nach einem der Ansprüche 5 bis 13 und einer medizinischen Werkzeugspitze (10) nach einem der Ansprüche 1 bis 4.

15. Verfahren (V) zur Befestigung einer medizinischen Werkzeugspitze (10) mit einem ersten Spitzenteil (20) und einem am ersten Spitzenteil (20) längsverschiebbar befestigten zweiten Spitzenteil (30) an einer Betätigungsvorrichtung (100) mit einem Grundkörper (120) und einem längsverschiebbar am Grundkörper (120) befestigten Schlitten (130), aufweisend die Schritte:
- (S1) Bringen des Schlittens (130) in eine Befestigungsstellung durch Längsverschieben relativ zum Grundkörper (120);
- (S2) werkzeugfreies Befestigen des ersten Spitzenteils (20) mithilfe eines ersten Befestigungsabschnitts (21) am Grundkörper (120) in einem Grundkörperbefestigungsabschnitt (121), wobei ein Einführen des ersten Befestigungsabschnitts (21) in den Grundkörperbefestigungsabschnitt (121) lediglich in der Befestigungsstellung möglich ist;
- (S3) werkzeugfreies Befestigen des zweiten Spitzenteils (30) mithilfe eines zweiten Befestigungsabschnitts (31) am Schlitten (130) in einem Schlittenbefestigungsabschnitt (131) durch Längsverschieben des Schlittens (130) relativ zum Grundkörper (120) aus der Befestigungsstellung heraus.

## Claims

1. A medical tool tip (10) for a surgical instrument (1), having
- a first tip part (20) with a first attachment portion (21) for toolless attachment of the first tip part (20) to a base body (120) of an actuation device (100), and
- a second tip part (30) with a second attachment portion (31) for toolless attachment of the second tip part (30) to a slide (130) of the actuation device (100), wherein the second tip part (30) is at least partially longitudinally displaceably attached to the first tip part (20), **characterized in that**
the first and second attachment portions (21, 31) are configured such that the second tip part (30) can be attached to the slide (130) by at least partial longitudinal displacement of the slide (130) only when the first tip part (20) has already been attached and secured to the base body (120).

2. The tool tip (10) as claimed in claim 1,
**characterized in that**
the first attachment portion (21) and the second attachment portion (31) have at least partially the same contour.

3. The tool tip (10) as claimed in any of the preceding claims,
**characterized in that**
- the first attachment portion (21) has a first protrusion (22) which, running longitudinally, adjoins a proximal end (23) of the first tip part (20) and has a top side (24) with a flat top side face (25),
- the second attachment portion (31) has a second protrusion (32) which, running longitudinally, adjoins a proximal end (33) of the second tip part (30) and has an underside (34) with a flat underside face (35) which is oriented substantially parallel to the top side face (25), and
- the first and second protrusions (22, 32) have respective side walls (26, 36) which run substantially perpendicularly to the top side face (25) and underside face (35) and are formed similarly uneven.

4. The tool tip (10) as claimed in any of the preceding claims,
**characterized in that**
- the first tip part (20) in the first attachment portion (21) has a top side (24) with a top side face (25) and a first bulge (27), which runs transversely to a surface normal (25a) of the top side face (25), and
- the second tip part (30) in the second attachment portion (31) has an underside (34) with an underside face (35) facing the top side face (25) and a second bulge (37), which runs transversely to a surface normal (35a) of the underside face (35) and is formed similarly to the first bulge (27).

5. An actuation device (100) for a surgical instrument (1), having
- a base body (120) with a base body attachment portion (121) for toolless attachment of a first tip part (20) of a medical tool tip (10) to the base body (120), and
- a slide (130) with a slide attachment portion (131) for toolless attachment of a second tip part (30) of the tool tip (10), which is longitudinally displaceably attached to the first tip part (20), to the slide (130), wherein the slide (130) is at least partially longitudinally displaceably attached to the base body (120),
**characterized in that**
for toolless attachment of the tool tip (10) to the actuation device (100), the slide (130)
- can be brought into an attachment position by at least partial longitudinal displacement relative to the base body (120), in which position the first tip part (20) can be attached to the base body attachment portion (121) of the base body (120), and
- is configured such that a first attachment portion (21) of the first tip part (20) can be inserted in the base body attachment portion (121) only in the attachment position, and, when the first tip part (20) is attached to the base body (120), the second tip part (30) can be attached to the slide (130) in the slide attachment portion (131) by at least partial longitudinal displacement of the slide (130) out of the attachment position.

6. The actuation device (100) as claimed in claim5,
**characterized in that**
a contour of the base body attachment portion (121) and a contour of the slide attachment portion (131) at least partially substantially align with one another when the slide (130) is in the attachment position.

7. The actuation device (100) as claimed in one of claims 5 or 6,
**characterized in that**
- the base body (120) has a base body top side (124) facing the slide (130) with a flat base body sliding face (125),
- the slide (130) has a slide underside (134) facing the base body (120) with a flat slide sliding face (135), via which the slide (130) at least partially slides along the base body sliding face (125) on a longitudinal displacement relative to the base body (120), and
- the base body sliding face (125) and the slide sliding face (135) at least partially substantially align with one another when the slide (130) is in the attachment position.

8. The actuation device (100) as claimed in any of claims 5 to 7,
**characterized in that**
the base body attachment portion (121) has a base body recess (122), and the slide attachment portion (131) has a slide recess (132), which at least partially substantially align with one another in the attachment position.

9. The actuation device (100) as claimed in any of claims 5 to 8,
**characterized in that**
the slide attachment portion (131) is configured to secure the first tip part (20) attached to the base body attachment portion (121).

10. The actuation device (100) as claimed in any of claims 5 to 9,
**characterized in that**
the slide (130) is configured to release the first tip part (20) attached to the base body attachment portion (121) in the attachment position.

11. The actuation device (100) as claimed in any of claims 5 to 10,
**characterized in that**
the slide (130) in the slide attachment portion (131) is formed as a fork for at least partially surrounding the second tip part (30).

12. The actuation device (100) as claimed in any of claims 5 to 11,
**characterized in that**
the slide attachment portion (131) has two fork tines (131a, 131b) which are designed to be deformable for attaching the second tip part (30) by longitudinal displacement of the slide (130).

13. The actuation device (100) as claimed in any of claims 5 to 12,
**characterized in that**
the slide attachment portion (131) is configured to engage on the second tip part (30) for attachment.

14. A surgical instrument (1) with an actuation device (100) as claimed in any of claims 5 to 13, and a medical tool tip (10) as claimed in any of claims 1 to 4.

15. A method (V) for attaching a medical tool tip (10) with a first tip part (20) and a second tip part (30), which is attached longitudinally displaceably on the first tip part (20), to an actuation device (100) with a base body (120) and a slide (130) which is longitudinally displaceably attached on the base body (120), having the steps:
- (S1) bringing the slide (130) into an attachment position by longitudinal displacement relative to the base body (120);
- (S2) toollessly attaching the first tip part (20) by means of a first attachment portion (21) on the base body (120) in a base body attachment portion (121), wherein the first attachment portion (21) can be inserted in the base body attachment portion (121) only in the attachment position;
- (S3) toollessly attaching the second tip part (30) by means of a second attachment portion (31) on the slide (130) in a slide attachment portion (131), by longitudinal displacement of the slide (130) relative to the base body (120) out of the attachment position.

## Revendications

1. Pointe d'outil médical (10) pour un instrument chirurgical (1), comportant
- une première partie de pointe (20) avec une première section de fixation (21) pour la fixation sans outil de la première partie de pointe (20) sur un corps de base (120) d'un dispositif d'actionnement (100), et
- une seconde partie de pointe (30) avec une seconde section de fixation (31) pour la fixation sans outil de la seconde partie de pointe (30) sur un chariot (130) du dispositif d'actionnement (100), la seconde partie de pointe (30) étant fixée au moins par endroits de manière à pouvoir coulisser longitudinalement sur la première partie de pointe (20),
**caractérisée en ce**
**que** la première et la seconde section de fixation (21, 31) sont formées de telle manière qu'une fixation de la seconde partie de pointe (30) sur le chariot (130) ne peut être effectuée par coulissement longitudinal au moins par endroits du chariot (130) que lorsque la première partie de pointe (20) est déjà fixée et bloquée sur le corps de base (120).

2. Pointe d'outil (10) selon la revendication 1,
**caractérisée en ce**
**que** la première section de fixation (21) et la seconde section de fixation (31) comportent au moins par endroits le même contour.

3. Pointe d'outil (10) selon l'une des revendications précédentes,
**caractérisée en ce**
**que**
- la première section de fixation (21) comporte une première partie faisant saillie (22), qui se raccorde en s'étendant longitudinalement à une extrémité proximale (23) de la première partie de pointe (20) et comporte un côté supérieur (24) avec une surface de côté supérieur (25) plane,
- la seconde section de fixation (31) comporte une deuxième partie faisant saillie (32), qui se raccorde en s'étendant longitudinalement à une extrémité proximale (33) de la seconde partie de pointe (30) et comporte un côté inférieur (34) avec une surface de côté inférieur (35) plane, qui est orientée sensiblement parallèlement à la surface de côté supérieur (25), et
- les première et deuxième parties faisant saillie (22, 32) comportent chacune des parois latérales (26, 36) qui s'étendent sensiblement perpendiculairement à la surface de côté supérieur (25) et à la surface de côté inférieur (35) et sont formées avec une irrégularité similaire.

4. Pointe d'outil (10) selon l'une des revendications précédentes,
**caractérisée en ce**
**que**
- la première partie de pointe (20) dans la première section de fixation (21) comporte un côté supérieur (24) avec une surface de côté supérieur (25) et un premier renflement (27), qui s'étend transversalement à une normale de surface (25a) de la surface de côté supérieur (25), et
- la seconde partie de pointe (30) dans la seconde section de fixation (31) comporte un côté inférieur (34) avec une surface de côté inférieur (35) tournée vers la surface de côté supérieur (25) et un second renflement (37), qui s'étend transversalement à une normale de surface (35a) de la surface de côté inférieur (35) et est formé de manière similaire au premier renflement (27).

5. Dispositif d'actionnement (100) pour un instrument chirurgical (1), comportant
- un corps de base (120) avec une section de fixation de corps de base (121) pour la fixation sans outil d'une première partie de pointe (20) d'une pointe d'outil médical (10) sur le corps de base (120) et
- un chariot (130) avec une section de fixation de chariot (131) pour la fixation sans outil d'une seconde partie de pointe (30) de la pointe d'outil (10), fixée de manière à pouvoir coulisser longitudinalement sur la première partie de pointe (130), sur le chariot (20), le chariot (130) étant fixé sur le corps de base (120) de manière à pouvoir coulisser longitudinalement au moins par endroits,
**caractérisé en ce**
**que**, pour la fixation sans outil de la pointe d'outil (10) sur le dispositif d'actionnement (100), le chariot (130)
- peut être amené par coulissement longitudinal au moins par endroits par rapport au corps de base (120) dans une position de fixation, dans laquelle la première partie de pointe (20) dans la section de fixation de corps de base (121) peut être fixée sur le corps de base (120), et
- est formé de telle manière qu'une introduction d'une première section de fixation (21) de la première partie de pointe (20) dans la section de fixation du corps de base (121) n'est possible que dans la position de fixation et la seconde partie de pointe (30) peut être reliée au chariot (130) dans la section de fixation de chariot (131) par coulissement longitudinal au moins par endroits du chariot (130) hors de la position de fixation lorsque la première partie de pointe (20) est fixée sur le corps de base (120).

6. Dispositif d'actionnement (100) selon la revendication 5,
**caractérisé en ce**
**qu'**un contour de la section de fixation de corps de base (121) et un contour de la section de fixation de chariot (131) sont alignés au moins par endroits et sensiblement l'un avec l'autre lorsque le chariot (130) se trouve dans la position de fixation.

7. Dispositif d'actionnement (100) selon l'une des revendications 5 ou 6,
**caractérisé en ce**
**que**
- le corps de base (120) comporte un côté supérieur de corps de base (124) tourné vers le chariot (130) avec une surface plane de glissement de corps de base (125),
- le chariot (130) comporte un côté inférieur de chariot (134) tourné vers le corps de base (120) avec une surface plane de glissement de chariot (135), avec laquelle le chariot (130) glisse au moins par endroits le long de la surface de glissement de corps de base (125) lors d'un coulissement longitudinal par rapport au corps de base (120), et
- la surface de glissement de corps de base (125) et la surface de glissement de chariot (135) sont alignées au moins par endroits et sensiblement l'une avec l'autre lorsque le chariot (130) se trouve dans la position de fixation.

8. Dispositif d'actionnement (100) selon l'une des revendications 5 à 7,
**caractérisé en ce**
**que** la section de fixation de corps de base (121) comporte un évidement de corps de base (122) et la section de fixation de chariot (131) comporte un évidement de chariot (132) qui, dans la position de fixation, sont alignés au moins par endroits et sensiblement l'un avec l'autre.

9. Dispositif d'actionnement (100) selon l'une des revendications 5 à 8,
**caractérisé en ce**
**que** la section de fixation de chariot (131) est mise au point pour bloquer la première partie de pointe (20) fixée sur la section de fixation de corps de base (121).

10. Dispositif d'actionnement (100) selon l'une des revendications 5 à 9,
**caractérisé en ce**
**que** le chariot (130) est mis au point pour libérer la première partie de pointe (20) fixée sur la section de fixation de corps de base (121) dans la position de fixation.

11. Dispositif d'actionnement (100) selon l'une des revendications 5 à 10,
**caractérisé en ce**
**que** le chariot (130) dans la section de fixation de chariot (131) est formé en fourche pour entourer au moins par endroits la seconde partie de pointe (30).

12. Dispositif d'actionnement (100) selon l'une des revendications 5 à 11,
**caractérisé en ce**
**que** la section de fixation de chariot (131) comporte deux bras de fourche (131a, 131b), qui sont formés de manière déformable pour fixer la seconde partie de pointe (30) par coulissement longitudinal du chariot (130).

13. Dispositif d'actionnement (100) selon l'une des revendications 5 à 12,
**caractérisé en ce**
**que** la section de fixation de chariot (131) est mise au point pour s'encliqueter sur la seconde partie de pointe (30) pour la fixation.

14. Instrument chirurgical (1) avec un dispositif d'actionnement (100) selon l'une des revendications 5 à 13 et une pointe d'outil médical (10) selon l'une des revendications 1 à 4.

15. Procédé (V) de fixation d'une pointe d'outil médical (10) avec une première partie de pointe (20) et une seconde partie de pointe (30) fixée de manière à pouvoir coulisser longitudinalement sur la première partie de pointe (20) sur un dispositif d'actionnement (100) avec un corps de base (120) et un chariot (130) fixé de manière à pouvoir coulisser longitudinalement sur le corps de base (120), comportant les étapes :
- (S1) amenée le chariot (130) dans une position de fixation par coulissement longitudinal par rapport au corps de base (120) ;
- (S2) fixation sans outil de la première partie de pointe (20) à l'aide d'une première section de fixation (21) sur le corps de base (120) dans une section de fixation de corps de base (121), une introduction de la première section de fixation (21) dans la section de fixation de corps de base (121) n'étant possible que dans la position de fixation ;
- (S3) fixation sans outil de la seconde partie de pointe (30) à l'aide d'une deuxième section de fixation (31) sur le chariot (130) dans une section de fixation de chariot (131) par coulissement longitudinal du chariot (130) par rapport au corps de base (120) hors de la position de fixation.
